# EUROPEAN PATENT APPLICATION

(11) **EP 2 574 338 A1**
(43) Date of publication of application: **03.04.2013**
(21) Application number: 10851812.7
(22) Date of filing: 20.05.2010
(51) Int. Cl.: A61K 31/19, A61K 31/195, A61K 31/66, A61P 17/00, A61P 31/04, A61P 31/10

(54) **SKIN PREPARATION COMPOSITION FOR EXTERNAL USE WITH EXCELLENT ANTIBACTERIAL AND ANTIFUNGAL EFFECTS**

(71) Applicant: Jung, Chung Hyun, Gwangju 506-010 (KR); Yang, Nam Woong, Gwangju 502-738 (KR)
(72) Inventor: Jung, Chung Hyun, Gwangju 506-010 (KR); Yang, Nam Woong, Gwangju 502-738 (KR)
(74) Representative: Keilitz, Wolfgang
(86) International application number: PCT/KR2010/003201
(87) International publication number: WO 2011/145765

(57) **Abstract**

The present invention relates to a skin preparation composition for external use with an excellent antibacterial effect, and more specifically, to a skin preparation composition for external use with excellent antibacterial and antifungal effects against bacteria and mold, comprising a first composition containing citric acid as an active ingredient and a second composition containing trisodium phosphate as an active ingredient.

## Description

### [Technical Field]

The present invention relates to a skin preparation composition for external use with an excellent antibacterial effect, and more specifically, to a skin preparation composition for external use with excellent antibacterial and antifungal effects against bacteria and fungi, comprising a first composition containing citric acid as an active ingredient and a second composition containing trisodium phosphate as an active ingredient.

### [Background Art]

Skin is a part of the body directly exposed to an outer environment and serves to control water evaporation and protect the body from external infection as well as protect important organs of the body as a protective layer. Due to coexisting *of Malassezia* fungi causing atopy with other bacteria such as *Staphylococcus aureus, Staphylococcus epidermidis* and *Propionibacterium acne* in the skin, when the fungi cause atopy, the bacteria worsen skin disorder caused by atopy. Therefore, it is needed to develop a skin preparation composition for external use having antibacterial effect for stopping bacteria from reproduction or sterilizing bacteria and antifungal effect for stopping fungi such as *Malassezia* from reproduction at the same time.

Citric acid is one of multibasic carboxylic acid having a hydroxyl group of chemical formula of C₆H₈O₇, and is contained in seeds or juice of many plants in free acid form. Citric acid fermentation is characterized by citric acid accumulation in a culture solution when microorganisms are cultured with saccharides as a substrate, and most of citric acid currently produced is made by this method. The citric acid may be added to foods or used as an analysis reagent or a blood coagulating agent.

As a conventional technique using the citric acid, Korean Patent Publication No. 2007-0002718 discloses a cosmetic composition containing a softened natural petal, wherein the citric acid is used to soften the natural petal. Korean Patent Publication No. 2005-0042199 discloses an organopolysiloxane polymer containing a glycerin derivative, characterized in that it can take up a liquid oil in an amount by weight not smaller than that of itself and be swollen therewith, and Korean Patent Publication No. 2007-0097647 discloses a shampoo composition for hair growing, characterized in that a raw material for hair growing is prepared by mixing *xanthium strumarium* fruit extract with swertia herb extract.

Further, Korean Patent Publication No.1999-0013555 discloses a cosmetic composition comprising: A) a cosmetically acceptable carrier; and B) 0.05 to 3.0 % by weight of a β-1,3-scleroglucan having a three-dimensional cross-linked triple helix structure based on the total weight of the composition.

### [Disclosure]

### [Technical Problem]

Therefore, the present inventors found that a skin preparation composition for external use, which has antibacterial effect for stopping bacteria from reproduction or sterilizing bacteria and antifungal effect for stopping fungi such as *Malassezia* from reproduction at the same time, can be prepared by separately storing citric acid and trisodium phosphate and mixing them together just before use.

Accordingly, an object of the present invention is to provide a skin preparation composition for external use with excellent antibacterial and antifungal effects.

### [Technical Solution]

In order to accomplish the aforementioned object, the present invention provides a skin preparation composition for external use with excellent antibacterial and antifungal effects, comprising a first composition containing citric acid as an active ingredient and a second composition containing trisodium phosphate as an active ingredient.

### [Advantageous Effects]

In the present invention, it was confirmed that when citric acid and trisodium phosphate were treated at the same time, growth of *Peptostreptococcus asaccharolyticus, Streptococcus mitis, Streptococcus mutans, S. salivarius, Streptococcus sanguinis, Pityrosporum ovale, Staphylococcus haemolyticus* and *Peptococcus niger* was significantly reduced. Accordingly, a skin preparation composition for external use with excellent antibacterial and antifungal effects, wherein citric acid and trisodium phosphate can be mixed together just before use, could be prepared.

### [Description of Drawings]

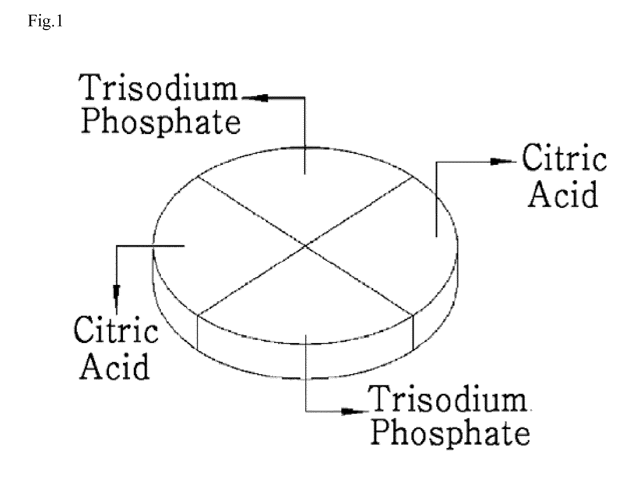

FIG. 1 is a perspective view of a soap formulation comprising a first composition containing citric acid and a second composition containing trisodium phosphate according to the present invention.

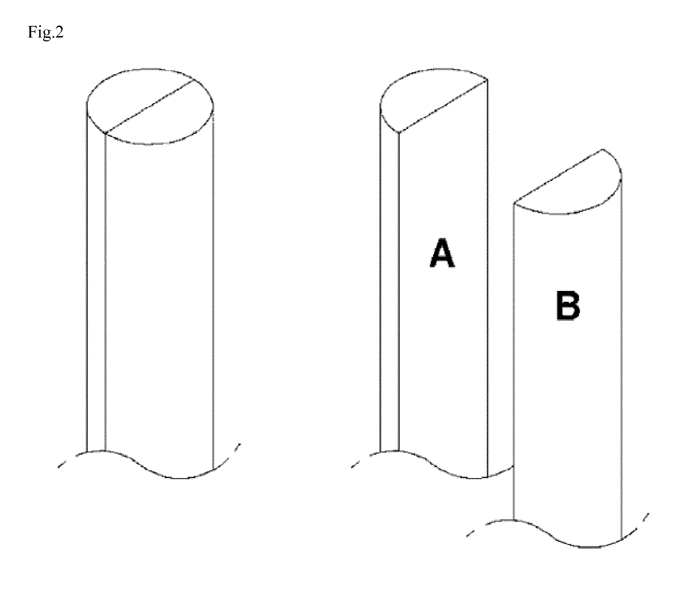

FIG. 2 is a perspective view of a lipstick formulation comprising a first composition containing citric acid and a second composition containing trisodium phosphate according to the present invention.

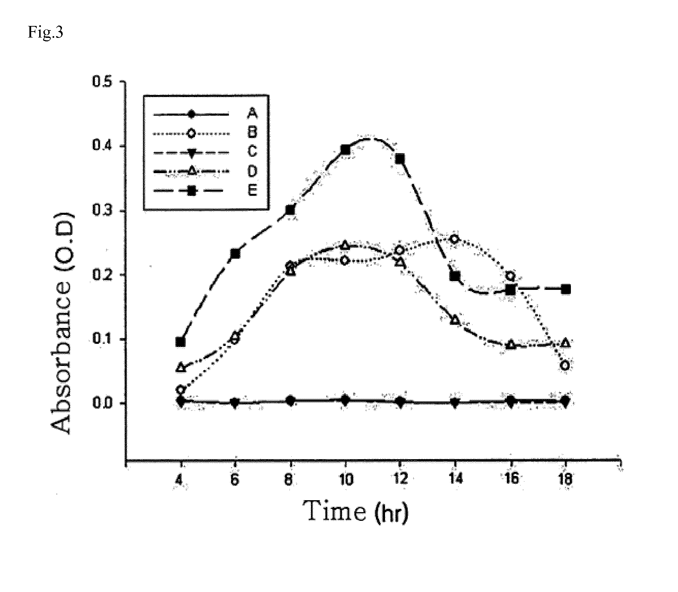

FIG. 3 is a graph showing the growth curve of *Streptococcus mitis* as the result of Test Example 2 [A: citric acid 0.4 g + trisodium phosphate 0.64 g (pH 6.0), B: trisodium phosphate 0.64 g + 5N HCl (pH 6.0), C: citric acid 0.4 g + 5N NaOH (pH 6.0), D: 5N HCl (pH 6.0) and E: broth control group (pH 7.3)].

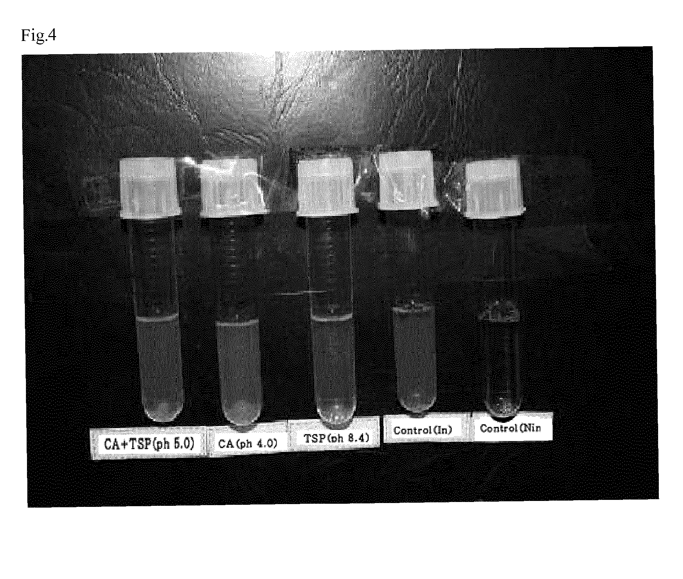

FIG. 4 is an image of the growth medium of *Pityrosporum ovale* as the result of Test Example 6 [from the left side of the image, citric acid+trisodium phosphate (pH 5.0), citric acid (pH 4.0), trisodium phosphate (TSP, pH 8.4), a control group (In) injected with only bacteria without a test compound, and a control group (Nin) containing only medium without injecting bacteria].

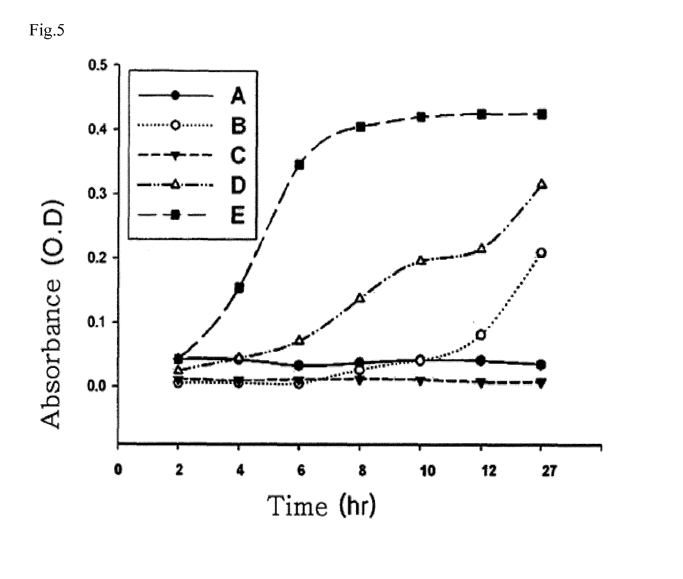

FIG. 5 is a graph showing the growth curve of *Staphylococcus haemolyticus* as the result of Test Example 7 [A: citric acid 8.0 mg + trisodium phosphate 20 mg/ml (pH 6.0), B: trisodium phosphate 20 mg/ml (pH 6.0), C: citric acid 8.0 mg + 5N NaOH (pH 6.0), D: 5N HCl (pH 6.0), E: broth control group (pH 7.3)].

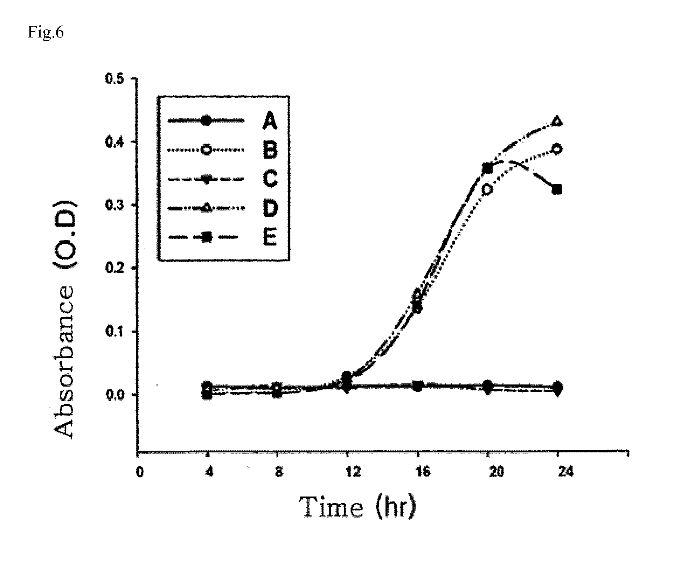

FIG. 6 is a graph showing the growth curve of *Peptococcus niger* as the result of Test Example 8 [A: citric acid 8.0 mg + trisodium phosphate 18.5 mg/ml (pH 6.0), B: trisodium phosphate 6.4 mg/ml (pH 6.0), C: citric acid 8.0 mg + 5N NaOH (pH 6.0), D: 5N HCl (pH 6.0), E: culture medium control group (pH 7.3)].

### [Best Mode]

Hereinafter, the present invention will be described in detail.

In the present invention, it was found that when citric acid having bacteriostatic or antibacterial effect against bacteria and trisodium phosphate having bacteriostatic effect against *Malassezia* are used at the same time, acidic and alkaline environments are formed at the same time in microenvironment of the skin, and the *Malassezia* is inhibited under micro alkaline environment and the bacteria are inhibited under micro acidic environment. Particularly, it was proved that citric acid has strong antibacterial or bacteriostatic effect, and also trisodium phosphate has bacteriostatic effect by removing mineral ingredients from the surrounding environment.

The present invention relates to a skin preparation composition for external use with excellent antibacterial and antifungal effects against bacteria and fungi, comprising a first composition containing citric acid as an active ingredient and a second composition containing trisodium phosphate as an active ingredient.

Preferably, the first composition may comprise citric acid in an amount of 0.1∼10 % by weight based on the total weight of the composition. If the amount of citric acid is less than 0.1 % by weight, it is difficult to obtain the desired antibacterial effect, and if the amount thereof is more than 10 % by weight, it may cause skin irritation or it is difficult to obtain formulation stability.

Preferably, the second composition may comprise trisodium phosphate in an amount of 0.1∼10 % by weight based on the total weight of the composition. If the amount of trisodium phosphate is less than 0.1 % by weight, it is difficult to obtain the desired antifungal effect, and if the amount thereof is more than 10 % by weight, it may cause skin irritation or it is difficult to obtain formulation stability.

Further, other ingredients of the first composition and the second composition except citric acid and trisodium phosphate may be the same, or different from each other according to the formulation, and may be easily selected from ingredients commonly used in the art by person skilled in the art.

In the skin preparation composition for external use according to the present invention, citric acid acts intensively on bacteria, and trisodium phosphate acts on fungi. Therefore, they respectively form an acidic environment and an alkaline environment at microenvironment of the skin due to separate actions of the two ingredients, and fungi are inhibited under micro alkaline environment and bacteria are inhibited under micro acidic environment when the two ingredients are used together just before use, rather than when they were mixed together in advance during preparation. Therefore, citric acid and trisodium phosphate should be stored separately and be mixed together just before use to obtain the best effect. To this end, the first composition and the second composition may be manufactured as products by being respectively packed in separate containers, or the first composition and the second composition may be encapsulated into microcapsules, nanocapsules, liposomes or the like, known in the art, so as to be mixed when using thereof, but not limited thereto.

The skin preparation composition for external use according to the present invention may be formulated to a cosmetic preparation such as a skin softener, a skin toner, a nourishing toner, a nourishing cream, a massage cream, an essence, an eye cream, an eye essence, a lotion, a lipstick, a powder, a baby powder, a body lotion, a body cream, a body oil and a body essence; a personal cleanser such as a soap, a cleansing cream, a cleansing lotion, a cleansing foam, a cleansing water and a body cleanser; a hair composition such as a hair nourishing toner, a hair essence, a hair serum, a scalp treatment, a hair treatment, a hair conditioner, a hair shampoo and a hair lotion; a tablet; and the like, but not limited thereto. Each of the formulations of the skin preparation composition for external use may comprise various ingredients mixed to common personal cleanser compositions according to its formulation or its final purpose, and kinds and amounts of the ingredients may be easily selected by a person skilled in the art.

In one embodiment of the present invention, the skin preparation composition for external use may be formulated to the form of a two-layered tablet prepared by combining a first layer comprising the first composition containing citric acid and a second layer comprising the second composition containing trisodium phosphate using a binder commonly used in the art. Further, a neutral toner, which has pH around 7.1 and does not irritate eyes, may be prepared by dissolving the two-layered tablet in water so that the citric acid contained in the first composition and the trisodium phosphate contained in the second composition are melted out at the same time, and the prepared toner may be used for washing face.

Further, in one embodiment of the present invention, as illustrated in FIG. 1, the skin preparation composition for external use may be formulated to a soap comprising the first composition and the second composition simultaneously by preparing a soap base containing the first composition and a soap base containing the second composition separately without mixing thereof together followed by combining them using a binder commonly used in the art.

Furthermore, in one embodiment of the present invention, as illustrated in FIG. 2, the skin preparation composition for external use may be formulated to a lipstick comprising the first composition and the second composition simultaneously by preparing a lipstick containing the first composition and a lipstick containing the second composition followed by combining them using a binder commonly used in the art.

Moreover, in one embodiment of the present invention, the skin preparation composition for external use may be formulated to a commercial baby powder or a commercial foot deodorizing powder, comprising the first composition and the second composition.

Further, in one embodiment of the present invention, the skin preparation composition for external use may be formulated to a personal cleanser composition such as a shampoo. Hereinafter, representative base ingredients and additives used for the shampoo composition will be described. For example, the shampoo composition may comprise a synthetic surfactant as an ingredient for cleansing, a preservative and water.

The synthetic surfactant used in the present invention may be any one selected from a group consisting of an anionic surfactant, an amphoteric surfactant and a nonionic surfactant. The synthetic surfactant may be used in an amount of 10∼70 % by weight, preferably 10∼30 % by weight, based on the total amount of the composition.

The synthetic anionic surfactant is alkyl and alkyl ether sulfate such as sodium lauryl sulfate, ammonium lauryl sulfate, triethanolamine lauryl sulfate, polyoxyethylene sodium lauryl sulfate and polyoxyethylene ammonium lauryl sulfate.

The synthetic amphoteric surfactant is alkyl betaine and alkyl amidopropyl betaine such as cocodimethyl carboxymethyl betaine, lauryldimethyl carboxymethyl betaine, lauryldimethyl alpha-carboxyethyl betaine, cetyldiemthyl carboxymethyl betaine and cocoamido propyl betaine.

The synthetic nonionic surfactant is alkanol amide and amine oxide such as lauryl diethyl amine oxide, palm oil alkyldimethyl amine oxide, lauric acid diethanlamide, palm oil fatty acid diethanolamide and palm oil fatty acid monoethanolamide.

In the present invention, the said surfactants may be used alone or in combination.

Optional ingredients, which may be used in the composition of the present invention, are common optional ingredients broadly known to those skilled in the art to maintain basic properties and quality. The optional ingredients may be a pearlescent agent, a preservative, a viscosity controlling agent, a pH controlling agent, a perfume, a hair conditioning agent and the like.

The pearlescent agent may be glycol distearate, glycol monostearate, fatty acid and the like, and it may be used in an amount of 0.1∼10 % by weight, preferably 1.0∼5.0 % by weight, based on the total amount of the composition.

The preservative may be methyl paraoxybenzoate, propyl paraoxybenzoate, sodium benzoate, methylchloroisothiazolinone, methylisothiazolinone and the like, and it may be used alone or in combination of two or more in an amount of 0.01∼5.0 % by weight, preferably or % by weight, based on the total amount of the composition.

The viscosity controlling agent may be an amide-based nonionic surfactant such as cocoamide MEA (CME), cocoamide DEA (CDE) and sodium chloride, and it may be used in an amount of 0.01∼10 % by weight, preferably 0.05∼5 % by weight, based on the total amount of the composition.

The pH controlling agent may be sodium phosphate, disodium phosphate, citric acid, sodium citrate and the like, and it may be used in an amount of 0.01∼5.0 % by weight, preferably 0.05∼2.0 % by weight, based on the total amount of the composition.

The hair conditioning agent may be dimethicone base, di-C₁₂₋₁₃ alkyl malate, cationic polymer and the like, and it may be used alone or in combination of two or more in an amount of 0.1∼10 % by weight, preferably 0.5∼5 % by weight, based on the total amount of the composition.

The shampoo composition according to the present invention comprising the said ingredients may be used according to common directions, and its frequency of use may be different depending on user's scalp condition or taste.

### [Mode for Invention]

Hereinafter, the following Test Examples and Formulation Examples are intended to further illustrate the present invention and thus are not limitative of the present invention. Accordingly, those skilled in the art will recognize that a variety of commonly known changes, substitutions and insertions of the embodiments described herein can be made and will be considered to fall in the scope of the invention.

[Test Example 1]

Growth of *Peptostreptococcus asaccharolyticus* (KTCC 3321) was observed in Wilkins Chalgren Anaerobe Broths containing citric acid 0.8 g, trisodium phosphate 1.85 g, 5N HCl and 5N NaOH according to the following prescriptions listed in Table 1, and the results thereof were shown in the following Table 1. The following control group is a broth not injected with the bacteria.

**Table 1**

| Absorbance (OD) | | | | | | |
|---|---|---|---|---|---|---|
| Time | Control | A | B | C | D | E |
| 4 hrs | 0.000 | 0.015 | 0.011 | 0.013 | 0.005 | 0.009 |
| 8 hrs | 0.000 | 0.010 | 0.007 | 0.010 | 0.010 | 0.010 |
| 12 hrs | 0.000 | 0.010 | 0.013 | 0.006 | 0.002 | 0.005 |
| 16 hrs | 0.000 | 0.014 | 0.012 | 0.009 | 0.008 | 0.007 |
| 20 hrs | | No change | Little growth | No change | No change | No change |
| 24 hrs | | No change | Little growth | No change | No change | No change |
| 56 hrs | | Clear | Full growth | Clear | Full growth | Full growth |
| A: Citric acid 0.8 g + Trisodium phosphate 1.85 g (pH 6.0) | | | | | | |
| B: Trisodium phosphate 1.85 g + 5N HCl (pH 6.0) | | | | | | |
| C: Citric acid 0.8 g + 5N NaOH (pH 6.0) | | | | | | |
| D: 5N HCl (pH 6.0) | | | | | | |
| E: Control group injected with only bacteria (pH 7.13) | | | | | | |

As shown in Table 1, growth of *P*. *asaccharolyticus* was completely suppressed up to 56 hrs in Broth A containing citric acid and trisodium phosphate and Broth C containing citric acid and NaOH.

[Test Example 2]

Growth of *Streptococcus mitis* (KCTC 3556) was observed in Brain Heart Infusion broths (Hereinafter, called "BHI broth") containing citric acid 0.4 g, trisodium phosphate 0.64 g, 5N HCl and 5N NaOH according to the following prescriptions listed in Table 2, and the results thereof were shown in the following Table 2 and FIG. 3. The following control group is a broth not injected with the bacteria.

**Table 2**

| Absorbance (OD) | | | | | | |
|---|---|---|---|---|---|---|
| Time | Control | A | B | C | D | E |
| 4 hrs | 0.000 | 0.004 | 0.019 | 0.002 | 0.054 | 0.095 |
| 6 hrs | 0.000 | 0.001 | 0.098 | 0.000 | 0.104 | 0.232 |
| 8 hrs | 0.000 | 0.003 | 0.213 | 0.003 | 0.205 | 0.300 |
| 10 hrs | 0.001 | 0.005 | 0.221 | 0.004 | 0.244 | 0.394 |
| 12 hrs | 0.000 | 0.002 | 0.236 | 0.001 | 0.218 | 0.380 |
| 14 hrs | 0.001 | 0.000 | 0.254 | 0.000 | 0.127 | 0.198 |
| 16 hrs | 0.000 | 0.003 | 0.197 | 0.001 | 0.089 | 0.176 |
| 18 hrs | 0.000 | 0.003 | 0.057 | 0.000 | 0.092 | 0.177 |
| 94 hrs | - | No growth | ND | No growth | ND | ND |
| A: Citric acid 0.4 g + Trisodium phosphate 0.64 g (pH 6.0) | | | | | | |
| B: Trisodium phosphate 0.64 g + 5N HCl (pH 6.0) | | | | | | |
| C: Citric acid 0.4 g + 5N NaOH (pH 6.0) | | | | | | |
| D: 5N HCl (pH 6.0) | | | | | | |
| E: Control group injected with only bacteria (pH 7.3) | | | | | | |

As shown in Table 2 and FIG. 3, strong bacteriostatic (or antibacterial) effect against *Streptococcus mitis* was confirmed in Broth A containing citric acid and trisodium phosphate and Broth C containing citric acid and NaOH.

[Test Example 3]

Growth of *Streptococcus mutans* (KCTC 3065) was observed in BHI broths containing citric acid 0.4 g, trisodium phosphate 0.64 g, 5N HCl and 5N NaOH according to the following prescriptions listed in Table 3, and the results thereof were shown in the following Table 3. The following control group is a broth not injected with the bacteria.

**Table 3**

| Absorbance (OD) | | | | | | |
|---|---|---|---|---|---|---|
| Time | Control | A | B | C | D | E |
| 4 hrs | 0.000 | 0.001 | 0.037 | 0.002 | 0.032 | 0.050 |
| 6 hrs | 0.000 | 0.003 | 0.069 | 0.002 | 0.092 | 0.157 |
| 8 hrs | 0.000 | 0.000 | 0.165 | 0.004 | 0.141 | 0.231 |
| 10 hrs | 0.000 | 0.002 | 0.198 | 0.003 | 0.200 | 0.307 |
| 12 hrs | 0.001 | 0.004 | 0.226 | 0.003 | 0.244 | 0.391 |
| 14 hrs | 0.001 | 0.005 | 0.235 | 0.001 | 0.212 | 0.372 |
| 16 hrs | 0.001 | 0.003 | 0.219 | 0.002 | 0.235 | 0.301 |
| 18 hrs | 0.001 | 0.002 | 0.212 | 0.001 | 0.210 | 0.232 |
| 94 hrs | - | No growth | ND | No growth | ND | ND |
| A: Citric acid 0.4 g + Trisodium phosphate 0.64 g (pH 6.0) | | | | | | |
| B: Trisodium phosphate 0.64 g + 5N HCI (pH 6.0) | | | | | | |
| C: Citric acid 0.4 g + 5N NaOH (pH 6.0) | | | | | | |
| D: 5N HCl (pH 6.0) | | | | | | |
| E: Control group injected with only bacteria (pH 7.3) | | | | | | |

As shown in Table 3, strong bacteriostatic (or antibacterial) effect against *Streptococcus mutans* was confirmed in Broth A containing citric acid and trisodium phosphate and Broth C containing citric acid and NaOH.

[Test Example 4]

Growth of *S. salivarius* (KCTC 3960) was observed in BHI broths containing citric acid 0.4 g, trisodium phosphate 0.64 g, 5N HCl and 5N NaOH according to the following prescriptions listed in Table 4, and the results thereof were shown in the following Table 4. The following control group is a broth not injected with the bacteria.

**Table 4**

| Absorbance (OD) | | | | | | |
|---|---|---|---|---|---|---|
| Time | Control | A | B | C | D | E |
| 4 hrs | 0.001 | -0.005 | 0.056 | -0.001 | 0.011 | 0.053 |
| 6 hrs | 0.001 | -0.006 | 0.198 | -0.001 | 0.068 | 0.102 |
| 8 hrs | 0.001 | -0.003 | 0.245 | -0.003 | 0.167 | 0.307 |
| 10 hrs | 0.001 | -0.003 | 0.260 | -0.004 | 0.192 | 0.315 |
| 12 hrs | 0.001 | -0.004 | 0.249 | -0.002 | 0.224 | 0.313 |
| 14 hrs | 0.000 | -0.005 | 0.243 | -0.004 | 0.234 | 0.268 |
| 16 hrs | 0.001 | -0.002 | 0.195 | -0.001 | 0.319 | 0.250 |
| 18 hrs | 0.001 | -0.003 | 0.177 | -0.004 | 0.224 | 0.232 |
| 94 hrs | - | No growth | ND | No growth | ND | ND |
| A: Citric acid 0.4 g + Trisodium phosphate 0.64 g (pH 6.0) | | | | | | |
| B: Trisodium phosphate 0.64 g + 5N HCl (pH 6.0) | | | | | | |
| C: Citric acid 0.4 g + 5N NaOH (pH 6.0) | | | | | | |
| D: 5N HCl (pH 6.0) | | | | | | |
| E: Control group injected with only bacteria (pH 7.3) | | | | | | |

As shown in Table 4, strong bacteriostatic (or antibacterial) effect against S. *salivarius* was confirmed in Broth A containing citric acid and trisodium phosphate and Broth C containing citric acid and NaOH.

[Test Example 5]

Growth of *Streptococcus sanguinis* was observed in BHI broths containing citric acid 0.4 g, trisodium phosphate 0.64 g, 5N HCl and 5N NaOH according to the following prescriptions listed in Table 5, and the results thereof were shown in the following Table 5. The following control group is a broth not injected with the bacteria.

**Table 5**

| Absorbance (OD) | | | | | | |
|---|---|---|---|---|---|---|
| Time | Control | A | B | C | D | E |
| 4 hrs | 0.000 | 0.003 | 0.055 | 0.004 | 0.018 | 0.060 |
| 6 hrs | 0.001 | 0.002 | 0.134 | 0.001 | 0.039 | 0.119 |
| 8 hrs | 0.000 | 0.000 | 0.182 | 0.002 | 0.118 | 0.196 |
| 10 hrs | 0.001 | 0.001 | 0.261 | 0.005 | 0.127 | 0.231 |
| 12 hrs | 0.000 | 0.003 | 0.282 | 0.003 | 0.283 | 0.313 |
| 14 hrs | 0.000 | 0.004 | 0.280 | 0.003 | 0.303 | 0.313 |
| 16 hrs | 0.001 | 0.003 | 0.251 | 0.001 | 0.203 | 0.246 |
| 18 hrs | 0.000 | 0.002 | 0.197 | 0.000 | 0.199 | 0.220 |
| 94 hrs | - | No growth | ND | No growth | ND | ND |
| A: Citric acid 0.4 g + Trisodium phosphate 0.64 g (pH 6.0) | | | | | | |
| B: Trisodium phosphate 0.64 g + 5N HCl (pH 6.0) | | | | | | |
| C: Citric acid 0.4 g + 5N NaOH (pH 6.0) | | | | | | |
| D: 5N HCl (pH 6.0) | | | | | | |
| E: Control group injected with only bacteria (pH 7.3) | | | | | | |

As shown in Table 5, strong bacteriostatic (or antibacterial) effect against S. *sanguinis* was confirmed in Broth A containing citric acid and trisodium phosphate and Broth C containing citric acid and NaOH.

[Test Example 6]

Each of citric acid (pH 4.0), trisodium phosphate (TSP, pH 8.4) and citric acid+trisodium phosphate (pH 5.0) was added to Leeming & Notman medium of 1 L containing glucose 10 g, peptone 10 g, bile salts 8 g, yeast extract 2 g, glycerol 1 monostearate 0.5 g, glycerol 10 ml and Tween 80 (1.5%) 15 ml, and 0.1 ml of *Pityrosporum ovale (Malassezia furfur),* which is a kind of fungi known for causing atopy, was injected to each group. The resulting medium was incubated at 32°C for 3 days and observed, and the results were shown in FIG. 4 [from the left side of the image, citric acid+trisodium phosphate (pH 5.0), citric acid (pH 4.0), trisodium phosphate (TSP, pH 8.4), control group (In) injected with only bacteria without test compounds, and control group (Nin) containing only medium without injecting bacteria].

As shown in FIG. 4, it was confirmed that growth of *P*. *ovale* was significantly suppressed in the medium containing trisodium phosphate at pH 8.0.

[Test Example 7]

Growth of *Staphylococcus haemolyticus,* which are isolated from human keratinocytes at a high rate and cause skin irritation, was observed in BHI broths respectively containing combinations of citric acid 8.0 mg, trisodium phosphate 20 mg/ml, 5N HCl and 5N NaOH, and the results were shown in FIG. 5 [A: citric acid 8.0 mg + trisodium phosphate 20 mg/ml (pH 6.0), B: trisodium phosphate 20 mg/ml (pH 6.0), C: citric acid 8.0 mg + 5N NaOH (pH 6.0), D: 5N HCl (pH 6.0), E: broth control group (pH 7.3)].

As shown in FIG. 5, it was confirmed that growth of S. *haemolyticus* was suppressed in Broth A containing citric acid and trisodium phosphate and Broth C containing citric acid and NaOH.

[Test Example 8]

Growth of *Peptococcus niger,* a kind of bacteria causing skin acne, was observed in BHI broths respectively containing combinations of citric acid 8.0 mg, trisodium phosphate, 5N HCl and 5N NaOH, and the results were shown in FIG. 6 [A: citric acid 8.0 mg + trisodium phosphate 18.5 mg/ml (pH 6.0), B: trisodium phosphate 6.4 mg/ml (pH 6.0), C: citric acid 8.0 mg + 5N NaOH (pH 6.0), D: 5N HCl (pH 6.0), E: broth control group (pH 7.3)].

As shown in FIG. 6, it was confirmed that growth of *P. niger* was suppressed in Broth A containing citric acid and trisodium phosphate and Broth C containing citric acid and NaOH.

From the results previously described, it was confirmed that when citric acid and trisodium phosphate are used at the same time, the optimal skin preparation composition for external use having the best antibacterial effect against bacteria and antifungal effect against fungi can be prepared.

[Formulation Example 1] Nourishing Toner

A nourishing toner of Formulation Example 1 was prepared by a conventional method according to the prescription listed in Table 6 (Unit: % by weight).

**Table 6**

| Ingredient | | Content |
|---|---|---|
| First Composition | Citric acid | 1.0 |
| | Squalane | 5.0 |
| | Beeswax | 4.0 |
| | Polysorbate 60 | 1.5 |
| | Sorbitan Sesquioleate | 1.5 |
| | Liquid paraffin | 0.5 |
| | Caprylic/Capric triglycerides | 5.0 |
| | Glycerin | 3.0 |
| | Butylene glycol | 3.0 |
| | Propylene glycol | 3.0 |
| | Carboxy vinyl polymer | 0.1 |
| | Triethanolamine | 0.2 |
| | Preservative, coloring, perfume | Appropriate amount |
| | Purified Water | Residual amount |
| Second Composition | Trisodium phosphate | 1.0 |
| | Squalane | 5.0 |
| | Beeswax | 4.0 |
| | Polysorbate 60 | 1.5 |
| | Sorbitan Sesquioleate | 1.5 |
| | Liquid paraffin | 0.5 |
| | Caprylic/Capric triglycerides | 5.0 |
| | Glycerin | 3.0 |
| | Butylene glycol | 3.0 |
| | Propylene glycol | 3.0 |
| | Carboxy vinyl polymer | 0.1 |
| | Triethanolamine | 0.2 |
| | Preservative, coloring, perfume | Appropriate Amount |
| | Purified Water | Residual amount |

[Formulation Example 2] Skin Softener

A skin softener of Formulation Example 2 was prepared by a conventional method according to the prescription listed in Table 7 (Unit: % by weight).

**Table 7**

| Ingredient | | Content |
|---|---|---|
| First Composition | Citric acid | 1.0 |
| | Glycerin | 3.0 |
| | Butylene glycol | 2.0 |
| | Propylene glycol | 2.0 |
| | Carboxy vinyl polymer | 0.1 |
| | PEG 12 nonylphenyl ether | 0.2 |
| | Polysorbate 80 | 0.4 |
| | Ethanol | 10.0 |
| | Triethanolamine | 0.1 |
| | Preservative, coloring, perfume | Appropriate amount |
| | Purified Water | Residual amount |
| Second Composition | Trisodium phosphate | 1.0 |
| | Glycerin | 3.0 |
| | Butylene glycol | 2.0 |
| | Propylene glycol | 2.0 |
| | Carboxy vinyl polymer | 0.1 |
| | PEG 12 nonylphenyl ether | 0.2 |
| | Polysorbate 80 | 0.4 |
| | Ethanol | 10.0 |
| | Triethanolamine | 0.1 |
| | Preservative, coloring, perfume | Appropriate Amount |
| | Purified Water | Residual amount |

[Formulation Example 3] Nourishing Cream

A nourishing cream of Formulation Example 3 was prepared by a conventional method according to the prescription listed in Table 8 (Unit: % by weight).

**Table 8**

| Ingredient | | Content |
|---|---|---|
| First Composition | Citric acid | 1.0 |
| | Polysorbate 60 | 1.5 |
| | Sorbitan Sesquioleate | 0.5 |
| | PEG 60 hydrogenated castor oil | 2.0 |
| | Liquid paraffin | 10.0 |
| | Squalane | 5.0 |
| | Caprylic/Capric triglycerides | 5.0 |
| | Glycerin | 5.0 |
| | Butylene glycol | 3.0 |
| | Propylene glycol | 3.0 |
| | Triethanolamine | 0.2 |
| | Preservative, coloring, perfume | Appropriate amount |
| | Purified Water | Residual amount |
| Second Composition | Trisodium phosphate | 1.0 |
| | Polysorbate 60 | 1.5 |
| | Sorbitan Sesquioleate | 0.5 |
| | PEG 60 hydrogenated castor oil | 2.0 |
| | Liquid paraffin | 10.0 |
| | Squalane | 5.0 |
| | Caprylic/Capric triglycerides | 5.0 |
| | Glycerin | 5.0 |
| | Butylene glycol | 3.0 |
| | Propylene glycol | 3.0 |
| | Triethanolamine | 0.2 |
| | Preservative, coloring, perfume | Appropriate amount |
| | Purified Water | Residual amount |

[Formulation Example 4] Massage Cream

A massage cream of Formulation Example 4 was prepared by a conventional method according to the prescription listed in Table 9 (Unit: % by weight).

**Table 9**

| Ingredient | | Content |
|---|---|---|
| First Composition | Citric acid | 1.0 |
| | Beeswax | 10.0 |
| | Polysorbate 60 | 1.5 |
| | Sorbitan Sesquioleate | 0.8 |
| | PEG 60 hydrogenated castor oil | 2.0 |
| | Liquid paraffin | 40.0 |
| | Squalane | 5.0 |
| | Caprylic/Capric triglycerides | 4.0 |
| | Glycerin | 5.0 |
| | Butylene glycol | 3.0 |
| | Propylene glycol | 3.0 |
| | Triethanolamine | 0.2 |
| | Preservative, coloring, perfume | Appropriate amount |
| | Purified Water | Residual amount |
| Second Composition | Trisodium phosphate | 1.0 |
| | Beeswax | 10.0 |
| | Polysorbate 60 | 1.5 |
| | Sorbitan Sesquioleate | 0.8 |
| | PEG 60 hydrogenated castor oil | 2.0 |
| | Liquid paraffin | 40.0 |
| | Squalane | 5.0 |
| | Caprylic/Capric triglycerides | 4.0 |
| | Glycerin | 5.0 |
| | Butylene glycol | 3.0 |
| | Propylene glycol | 3.0 |
| | Triethanolamine | 0.2 |
| | Preservative, coloring, perfume | Appropriate amount |
| | Purified Water | Residual amount |

[Formulation Example 5] Lipstick Formulation

A lipstick of Formulation Example 5 was prepared by a conventional method according to the prescription listed in Table 10 (Unit: % by weight).

**Table 10**

| Ingredient | | Content |
|---|---|---|
| First Composition | Citric acid | 1.0 |
| | Candelilla wax | 7.0 |
| | Beeswax | 5.0 |
| | Polyglyceryl-2-triisostearate | 25.0 |
| | Castor Oil | Residual amount |
| | Hydrogenated polyisobutene | 10.0 |
| | C10-11 isoparaffin | 2.0 |
| | Silicone gel | 1.0 |
| | Vitamin E acetate | 2.0 |
| | Titanium dioxide | 3.0 |
| | Iron oxide | 5.3 |
| | FD & C yellow #5 aluminum-lake | 4.5 |
| | D & C red #7 calcium-lake | 1.2 |
| | Talc | 4.0 |
| | Silica | 5.0 |
| | Preservative | Appropriate amount |
| | Perfume | 0.2 |
| Second Composition | Trisodium phosphate | 1.0 |
| | Candelilla wax | 7.0 |
| | Beeswax | 5.0 |
| | Polyglyceryl-2-triisostearate | 25.0 |
| | Castor Oil | Residual amount |
| | Hydrogenated polyisobutene | 10.0 |
| | C10-11 isoparaffin | 2.0 |
| | Silicone gel | 1.0 |
| | Vitamin E acetate | 2.0 |
| | Titanium dioxide | 3.0 |
| | Iron oxide | 5.3 |
| | FD & C yellow #5 aluminum-lake | 4.5 |
| | D & C red #7 calcium-lake | 1.2 |
| | Talc | 4.0 |
| | Silica | 5.0 |
| | Preservative | Appropriate amount |
| | Perfume | 0.2 |

Preparation Method

(1) The candelilla wax and the beeswax were weighed according to each weight ratio, and heated to 80∼90°C for melting.

(2) The polyglyceryl-2-triisostearate, the castor oil, the hydrogenated polyisobutene, the C₁₀₋₁₁ isoparaffin, the silicone gel and the vitamin E acetate, weighed according to each weight ratio, were added to the melted mixture prepared in (1), and the resulting mixture was heated to 75∼80°C and stirred for melting.

(3) The titanium dioxide, the iron oxide, the FD & C yellow #5 aluminum-lake, the D&C red #7 calcium-lake, the talc, the silica and the preservative, weighed according to each weight ratio, were added to the melted mixture prepared in (2), and dispersed therein using a homo mixer.

(4) The resulting mixture of (3) was deaerated, and the perfume was added thereto. The resulting mixture was poured into a mold such as a metal mold or a disposable plastic mold at 80∼90°C to respectively mold the first composition and the second composition, and then the molded compositions were combined together using a binder to obtain a lipstick comprising the first composition and the second composition (FIG. 2).

[Formulation Example 6] Shampoo Formulation

A shampoo of Formulation Example 6 was prepared by a conventional method according to the prescription listed in Table 11 (Unit: % by weight).

**Table 11**

| Ingredient | | Content |
|---|---|---|
| First Composition | Citric acid | 1.0 |
| | Ammonium lauryl sulfate | 10.0 |
| | Polyoxyethylene ammonium lauryl sulfate | 5.0 |
| | Cocoamidopropyl Betaine | 2.0 |
| | Trihydroxystearin | 0.15 |
| | Cocoyl monoethanolamide | 0.8 |
| | Guar Hydroxypropyltrimonium Chloride | 0.2 |
| | Methyl paraben | 0.1 |
| | Preservative, coloring, perfume | Appropriate amount |
| | Dimethicone | 1.0 |
| | Purified Water | Residual amount |
| Second Composition | Trisodium phosphate | 1.0 |
| | Ammonium lauryl sulfate | 10.0 |
| | Polyoxyethylene ammonium lauryl sulfate | 5.0 |
| | Cocoamidopropyl Betaine | 2.0 |
| | Trihydroxystearin | 0.15 |
| | Cocoyl monoethanolamide | 0.8 |
| | Guar Hydroxypropyltrimonium Chloride | 0.2 |
| | Methyl paraben | 0.1 |
| | Preservative, coloring, perfume | Appropriate amount |
| | Dimethicone | 1.0 |
| | Purified Water | Residual amount |

[Formulation Example 7] Preparation of Tablet

Citric acid 100 mg, lactose 400 mg, corn starch 400 mg and magnesium stearate 2 mg were mixed together followed by tableting them using a tableting machine according to a conventional method for preparing a tablet, obtaining a tablet containing citric acid.

Trisodium phosphate 100 mg, lactose 400 mg, corn starch 400 mg and magnesium stearate 2 mg were mixed together followed by tableting them using a tableting machine according to a conventional method for preparing a tablet, obtaining a tablet containing trisodium phosphate. Then, the tablet containing trisodium phosphate was combined with the tablet containing citric acid using a binder to obtain a two-layered tablet.

[Formulation Example 8] Preparation of Toner

The tablet prepared in Formulation Example 7 10 g was dissolved in water 990 cc to obtain a neutral toner, which has pH around 7.1 and does not irritate eyes. The toner can be used for washing face.

[Formulation Example 9] Preparation of Soap

Citric acid 5 g, palm oil fatty acid 40 ml and palm kernel fatty acid 20 ml were put into a reaction tank and heated to 80°C while maintaining a pedal at around 100 RPM. When the temperature of the content became 80°C, sodium hydroxide solution 17 ml was slowly added thereto. When the sodium hydroxide solution was added to the fatty acid solution, soap was slowly formed, and the temperature of the content increased to 90°C or more due to neutralization reaction of soap. At the beginning of sodium hydroxide addition, the pedal was maintained at 150 RPM to prevent coagulation of the content. The resulting content was dried to obtain a soap containing citric acid.

The method described above was repeated except for using trisodium phosphate 5 g instead of citric acid to obtain a soap containing trisodium phosphate, and then the soap was combined with the soap containing citric acid using a binder to obtain a two-layered soap.

## Claims

1. A skin preparation composition for external use with antibacterial and antifungal effects, comprising:
a first composition containing citric acid as an active ingredient; and
a second composition containing trisodium phosphate as an active ingredient.

2. The skin preparation composition for external use according to claim 1, wherein the first composition comprises citric acid in an amount of 0.1∼10 % by weight based on the total weight of the composition.

3. The skin preparation composition for external use according to claim 2, wherein the second composition comprises trisodium phosphate in an amount of 0.1∼10 % by weight based on the total weight of the composition.

4. The skin preparation composition for external use according to claim 3, wherein the skin preparation composition is formulated to a cosmetic preparation selected from the group consisting of a skin softener, a skin toner, a nourishing toner, a nourishing cream, a massage cream, an essence, an eye cream, an eye essence, a lotion, a lipstick, a powder, a baby powder, a body lotion, a body cream, a body oil and a body essence.

5. The skin preparation composition for external use according to claim 3, wherein the skin preparation composition is formulated to a personal cleanser selected from the group consisting of a soap, a cleansing cream, a cleansing lotion, a cleansing foam, a cleansing water and a body cleanser.

6. The skin preparation composition for external use according to claim 3, wherein the skin preparation composition is formulated to a hair composition selected from the group consisting of a hair nourishing toner, a hair essence, a hair serum, a scalp treatment, a hair treatment, a hair conditioner, a hair shampoo and a hair lotion.

7. The skin preparation composition for external use according to any one of claims 1 to 6, wherein the skin preparation composition is formulated to a two-layered tablet comprising a first layer containing citric acid and a second layer containing trisodium phosphate.

8. The skin preparation composition for external use according to claim 7, wherein the skin preparation composition is a neutral toner of pH 7.1 prepared by dissolving the two-layered tablet in water.
